# EUROPÄISCHE PATENTSCHRIFT

(11) **EP 2 139 403 B1**
(45) Veröffentlichungstag und Bekanntmachung des Hinweises auf die Patenterteilung: **14.07.2010**
(21) Anmeldenummer: 08714316.0
(22) Anmeldetag: 04.04.2008
(51) Int. Cl.: A61B 17/02, A61B 1/31, A61M 29/00, A61B 17/12

(54) **EINRICHTUNG ZUR VERWENDUNG BEI DER BEHANDLUNG EINES HÄMORRHOIDENPROLAPS**
DEVICE FOR USE FOR THE TREATMENT OF HEMORRHOID PROLAPSE
DISPOSITIF DESTINÉ AU TRAITEMENT D'UN PROLAPSUS HÉMORROÏDAIRE

(30) Priorität: 23.04.2007 AT 6322007
(43) Veröffentlichungstag der Anmeldung: 06.01.2010
(73) Patentinhaber: A.M.I. Agency for Medical Innovations GmbH, 6800 Feldkirch (AT)
(72) Erfinder: SZINICZ, Gerhard, A-6900 Bregenz (AT)
(74) Vertreter: Hofmann, Ralf U.
(86) Internationale Anmeldenummer: PCT/AT2008/000123
(87) Internationale Veröffentlichungsnummer: WO 2008/128261

(56) Entgegenhaltungen:
- EP-A- 1 683 473
- WO-A-2007/016946

## Beschreibung

Die vorliegende Erfindung betrifft eine Einrichtung zur Verwendung bei der Behandlung eines Hämorrhoidenprolaps, insbesondere durch HAL-Operation und/oder Anbringen einer Raffungsnaht und/oder einer Gummibandligatur, mit einem Tubus mit einer Tubusmantelwand und einer Verschlusseinrichtung mit einer einen Hohlraum umschließenden Verschlussmantelwand, wobei die Tubusmantelwand im Hohlraum der Verschlussmantelwand verschiebbar und/oder drehbar lagerbar ist und die Tubusmantelwand und die Verschlussmantelwand_ einzeln oder gemeinsam in ein Rektum eines Patienten einführbar sind, wobei die Tubusmaritelwand zumindest eine Tubusmantelöffnung und die Verschlussmantelwand zumindest eine Verschlussmantelöffnung aufweisen und die Tubusmantelöffnung und die Verschlussmantelöffnung in zumindest einer Öffnungsstellung zumindest teilweise miteinander in Deckung bringbar sind.

Bekannt ist bereits die Durchführung einer minimal-invasiven Hämorrhoiden-Arterienligatur mittels einer in das Rektum einzuführenden Ultraschallsonde. Im distalen Bereich eines Tubus ist ein Ultraschallsensor angeordnet, und zwar neben einer Öffnung in der Mantelwand des Tubus. Mittels des Ultraschallsensors wird eine innerhalb der Darmwand verlaufende Hämorrhoiden-Arterie lokalisiert, wobei in der Folge eine Ligatur bzw. eine HAL-Operation der Arterie durch die Öffnung im Tubus erfolgt. Ein Vorteil dieser heute häufig angewendeten Behandlungsmethode ist neben der minimalen Invasivität, dass die Behandlung im Bereich der Darmwand oberhalb der Linea Dentata erfolgt und somit nicht in einem schmerzempfindlichen Bereich. Allerdings kann diese Methode bei einem Hämorrhoidenprolaps des III und IV Grades häufig nicht mehr den gewünschten Behandlungserfolg bringen. Es handelt sich hierbei um ausgeprägte prolabierende Hämorrhoidalpolster (= prolabierende Noduli) bzw. prolabierende Mukosa, die mehr oder weniger ausgeprägt und mehr oder weniger permanent aus dem Anus prolabieren. Derartige Einrichtungen zur Hämorrhoiden-Arterienligatur sind beispielsweise aus der US 5,570,692 A und der EP 1 234 539 A2 bekannt. Letztgenannte Schrift zeigt in Fig. 12 auch eine sogenannte Gummibandligatur.

Es wurden weiters vereinzelt bereits Operationen durchgeführt, bei welchen die prolabierenden Noduli mittels Raffungsnähten gerafft wurden, um den Prolaps wesentlich zu verringern. Diese Operationen haben sich jedoch als technisch schwierig erwiesen, da je nach Befund der Prolaps der Schleimhaut zielgerechte Durchstechungen in diesem Bereich verunmöglicht, die Einhaltung eines ausreichenden Abstands zur Linea Dentata nicht ausreichend kontrollierbar ist (Schmerzen sind die Folge) und der Zeitaufwand für die Einzeldurchstechungen zu groß ist (vier bis fünf Durchstechungen sind meist erforderlich, um zum gewünschten Erfolg zu gelangen).

Eine gattungsgemäße Einrichtung zur Verbesserung dieser Operationsmethode ist bereits aus der EP 1 683 473 A1 bekannt. Bei der dort vorgeschlagenen Einrichtung kann durch sukzessives Verdrehen der Verschlusseinrichtung gegen den Tubus ein Operationsspalt, durch den hindurch die Mukosa erreichbar ist, sukzessive vom distalen Ende der Einrichtung beginnend vergrößert werden. Der Operationsspalt entsteht durch das zumindest teilweise in Deckung miteinander bringen der Tubusmantelöffnung mit der Verschlussmantelöffnung. Allerdings hat sich in der Praxis herausgestellt, dass die zwischen Tubusmantelöffnung und Verschlussmantelöffnung immer größer werdende effektive Öffnung des Operationsspalts zu groß wird, sodass die Mukosa zu weit in das Innere des Tubus eindringt.

Eine zur EP 1 683 473 A1 von der Grundstruktur her ähnliche Einrichtung ist in der US 295,798 gezeigt. Hier ist die Tubusmantelöffnung stufenweise vergrößert. Auch hier ist daher mit einem zu weiten Eindringen der Mukosa in das Innere des Tubus zu rechnen.

Aufgabe der Erfindung ist es, eine gattungsgemäße Einrichtung dahingehend zu verbessern, dass ein zu weites Eindringen der Mukosa in das Innere des Tubus verhindert ist.

Dies gelingt durch eine Einrichtung mit den Merkmalen des Patentanspruchs 1.

Der oder die Stege gewähren einerseits nach wie vor die Möglichkeit, aus dem Inneren des Tubus mit entsprechenden an sich bekannten Operationsinstrumenten durch die Tubusmantelöffnung und die Verschlussmantelöffnung hindurch zur zu operierenden Mukosa zu gelangen. Andererseits stützen der oder die Stege die Mukosa aber in der Form ab, dass ein zu tiefes Eindringen der Mukosa in das Innere des Tubus verhindert ist. Der oder die Stege teilen somit die Operationsöffnung bzw. das Operationsfenster in, vorzugsweise in Längsrichtung des Tubus hintereinander angeordnete, Einzelöffnungen durch die hindurch operiert werden kann.

Bevorzugt ist dabei vorgesehen, dass die Einrichtung vor der Operation in einer Schließstellung, in der sowohl Verschlussmantelöffnung als auch Tubusmantelöffnung geschlossen sind, in das Rektum des Patienten einführbar ist. Erst kurz vor der Operation wird, vorzugsweise durch Verdrehen der Verschlusseinrichtung gegen den Tubus, ein Operationsfenster durch die in Deckung gebrachte Tubusmantelöffnung und Verschlussmantelöffnung hindurch geöffnet. In dieser sogenannten Öffnungsstellung bleiben die erfindungsgemäßen Stege im Operationsfenster, wodurch die Mukosa während der Operation abgestützt wird. Um das Verdrehen der Verschlusseinrichtung gegen den Tubus zu ermöglichen, ist günstigerweise vorgesehen, dass die Tubusmantelwand und/oder die Verschlussmantelwand abgesehen von darin vorgesehenen Öffnungen zumindest bereichsweise rotationssymmetrisch bezüglich einer Längsachse, vorzugsweise zylinder- oder kegel- oder kegelstumpfförmig, ausgebildet ist (sind).

Durch die Erfindung kann somit eine sehr universell einsetzbare Einrichtung geschaffen werden, mit der eine an sich bekannte sogenannte HAL- bzw. Ligaturoperation wie auch das Anbringen einer Raffungsnaht aber auch eine Gummibandligatur möglich ist. Insbesondere für die Schaffung einer Raffungsnaht ist es günstig, wenn die Tubusmantelöffnung und/oder die Verschlussmantelöffnung zwischen ihrem distalen Ende und ihrem proximalen Ende längserstreckt ist (sind).

Im Sinne einer optimalen Abstützung der Mukosa während der Operation ist es günstig, wenn ein jeweiliger Steg beabstandet vom distalen Ende und proximalen Ende der Tubusmantelöffnung und/oder der Verschlussmantelöffnung angeordnet ist.

Im Falle mehrer Stege ist bevorzugt, wenn jeweils zwei benachbarte Stege, vorzugsweise in Längsrichtung der Tubusmantelwand und/oder der Verschlussmantelwand, voneinander beabstandet und/oder parallel zueinander verlaufend angeordnet sind.

Es sind grundsätzlich verschiedene Arten der Ausformung und Anordnung der Stege möglich. Bevorzugt ist aber z. B. wenn ein jeweiliger Steg längserstreckt ist und seine Längserstreckung im Wesentlichen senkrecht zu einer Längsachse der Tubusmantelwand und/oder der Verschlussmantelwand verlaufen. Unter im Wesentlichen senkrecht ist vorzugsweise ein Winkel zwischen 70° und 110°, ganz bevorzugt zwischen 80° und 100°, zu verstehen. Unter einer Längserstreckung wird allgemein verstanden, dass die Ausdehnung eines Gegenstands in Längsrichtung deutlich größer als die Ausdehnung in Querrichtung, also vorzugsweise mindestens das Doppelte der Erstreckung in einer Querrichtung, ist.

Die Begriffe distal und proximal sind in Bezug auf die erfindungsgemäße Einrichtung so zu verstehen, dass sie auf die Seite bezogen sind, die dem Operateur zugewandt ist. Somit ist das distale Ende der Einrichtung das Einführende des Tubus und das proximale Ende das gegenüberliegende Ende der Einrichtung.

Weitere Ausgestaltungsformen und Merkmale der Erfindung werden anhand eines in den Fig. dargestellten bevorzugten Ausführungsbeispiels der Erfindung erläutert.
Dabei zeigen:
- die Fig. 1a und 1b: den Tubus und die Verschlusseinrichtung des Ausführungsbeispiel in einer Explosionsdarstellung;
- Fig. 2: eine Ansicht auf das proximale Ende der Einrichtung;
- Fig. 3: die Schließstellung der Einrichtung, welche beim Einführen in das Rektum des Pa- tienten gewählt wird;
- Fig. 4: die Öffnungsstellung in der eine Raffungsnaht an der Mukosä angebracht wird;
- Fig. 4a: eine Ansicht von Innen auf das Operationsfenster;
- Fig. 5: die Stellung der Verschlusseinrichtung kurz vor Fertigstellung der Raffungsnaht;
- Fig. 5a: eine entsprechend schematisierte Ansicht auf die Mukosa aus dem Innenhohlraum des Tubus heraus und
- die Fig. 6a bis 7: eine spezielle Verwendungsform des Tubus der Einrichtung zur Durchfüh- rung einer Gummibandligatur.

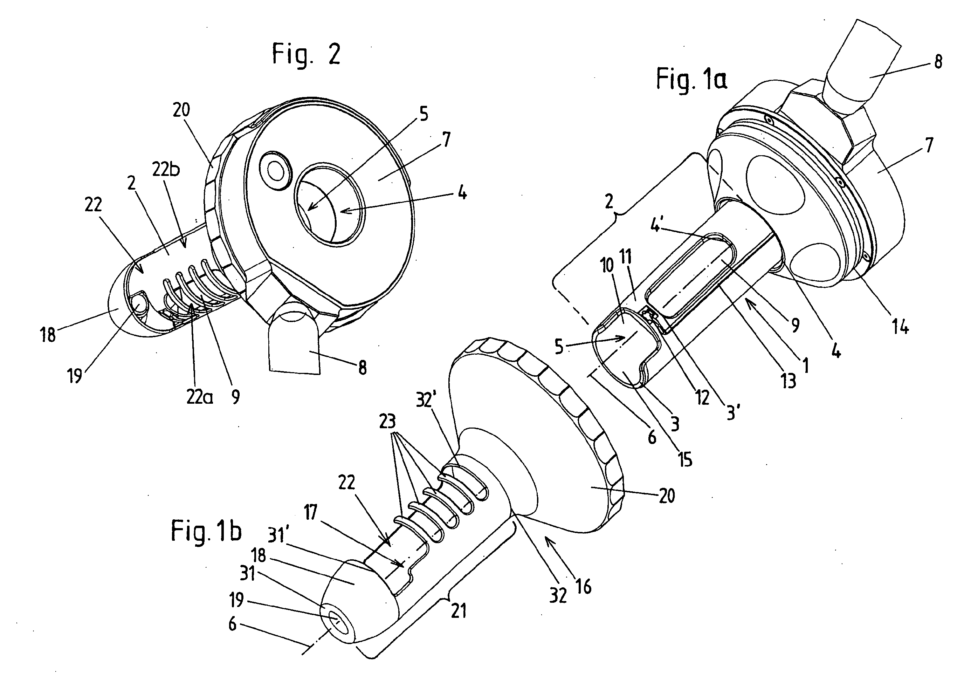

Fig. 1a zeigt zunächst einen an der Halterung 7 befestigten Tubus 1. Die Halterung 7 trägt wie an sich bekannt einen Handgriff 8. Der Tubus 1 weist eine bezüglich der Längsachse 6 - abgesehen von der Tubusmantelöffnung 9 und der Ligaturöffnung 10 - rotationssymmetrische Tubusmantelwand 2 auf. Diese erstreckt sich von ihrem distalen Ende 3 zu ihrem proximalen Ende 4. Zwischen der ebenfalls in Richtung der Längsachse 6 längserstreckten Tubusmantelöffnung 9 und der dazu distal angeordneten Ligaturöffnung 5 ist auf einem Zwischensteg 11 der Tubusmantelwand 2 ein an sich bekannter Ultraschallsensor 12 angeordnet. Seine Versorgungsleitungen sind in dem Kanal 13 verborgen. Der Ultraschallsensor 12 dient zur Lokalisierung einer Arterie. Je nach Ausrichtung des Ultraschallsensors 12 kann er diese im Bereich der Ligaturöffnung 10 oder der Tubusmantelöffnung 9 orten.

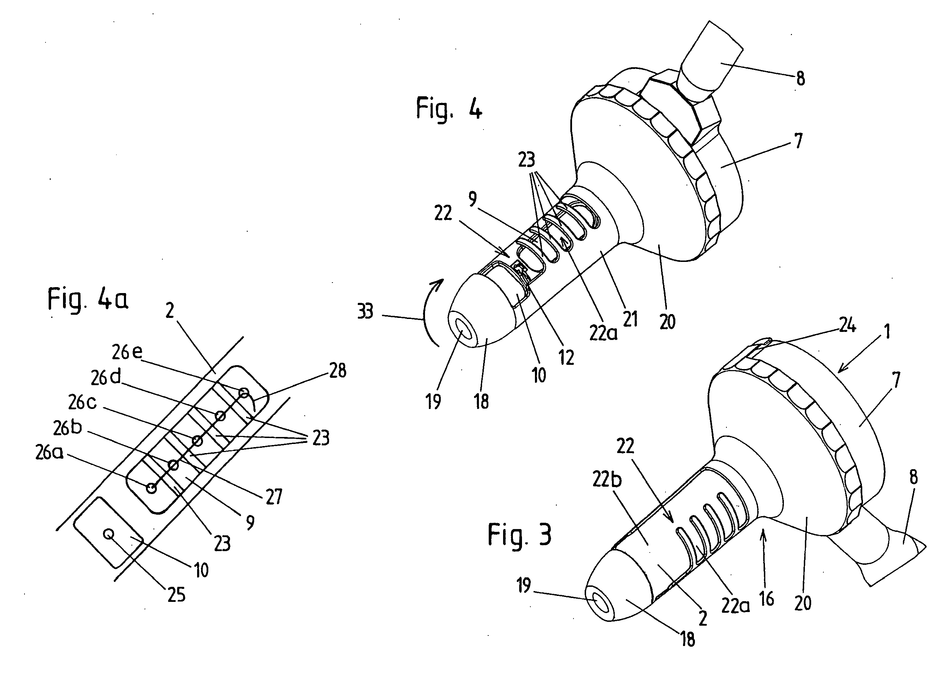

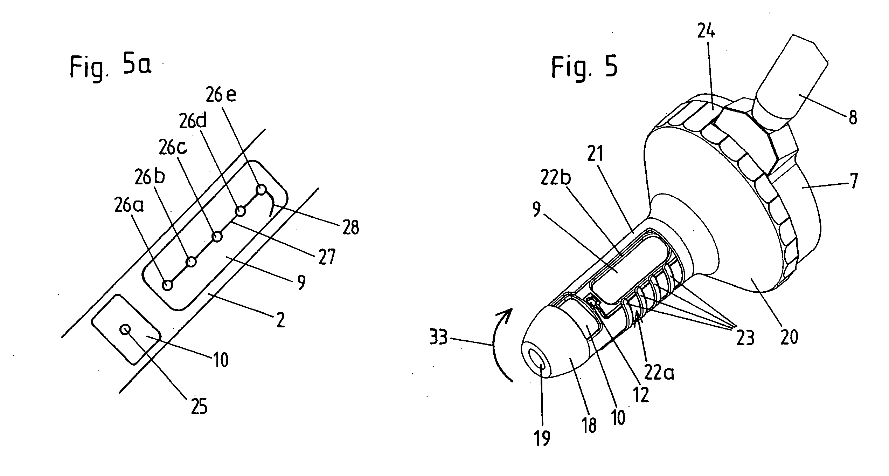

Wie weiter unten anhand der Fig. 4a und 5a gezeigt, dient bei diesem bevorzugten Ausführungsbeispiel die Ligaturöffnung 10 zur Durchführung der an sich bekannten HAL-Operation während die Raffnaht durch die zwischen ihrem distalen Ende 3' und ihrem proximalen Ende 4' längserstreckte Tubusmantelöffnung 9 hindurch an der Mukosa angebracht werden kann. Dies muss aber nicht zwingend so vorgesehen sein. Es ist durchaus denkbar, auf die Ligaturöffnung 10 zu verzichten und die Hall-Operation im distalen Endbereich der Tubusmantelöffnung 9 durchzuführen. Bei dieser Ausgestaltungsform kann die Tubusmantelöffnung 9 entsprechend lang ausgeführt sein und der Ultraschallsensor 12 die Arterie im Bereich der Tubusmantelöffnung 9 orten.

Fig. 1b zeigt die Verschlusseinrichtung 16 dieses Ausführungsbeispiels in deren Hohlraum 17 die Tubusmantelwand 2 eingeführt werden kann. Dabei ist bevorzugt vorgesehen, dass die Gestalt der Außenfläche der Tubusmantelwand 2 so exakt mit der Gestalt der Innenfläche der Verschlussmantelwand 21 übereinstimmt, dass bei Einführen der Tubusmantelwand 2 in den Hohlraum 17 der Verschlussmantelwand 21, bis auf das zum Einführen bzw. Drehen benötigte Spiel, ein Passsitz erzielbar ist.

Die Verschlusseinrichtung 16 weist zur exakten Führung einen Führungsabschnitt 20 auf, welcher in der Montagestellung gemäß der Fig. 2 bis 5 im Eingriff mit der Führung 14 der Halterung 7 des Tubus 1 steht. Die die Tubusmantelwand 2 aufnehmende Verschlussmantelwand 21 ist zumindest in ihrem mittleren und proximalen Bereich abgesehen von der Verschlussmantelöffnung 22 - wie auch die Tubusmantelwand 2 - bezüglich der Längsachse 6 rotationssysmmetrisch und im Wesentlichen zylinderförmig ausgeführt. Ihr distales Ende ist mit 31, ihr proximales Ende mit 32 bezeichnet. Der distale Abschnitt 18 ist, um das Einführen in das Rektum des Patienten zu erleichtern, konisch abgerundet. Tubusmantelwand 2 wie auch Verschlussmantelwand 21 weisen in diesem Ausführungsbeispiel ein distal offenes Ende auf. Durch die entsprechenden Öffnungen 15 und 19 ist es für den Chirurgen während der Operation möglich, ein Instrument durch das offene distale Ende der Einrichtung hindurchzuführen, um mit diesem Instrument prolabierte Schleimhaut in die distale Richtung zu drücken, z. b. um eine Raffung zu unterstützen und die anzubringende Naht zu entlasten. Eine am distalen Ende geschlossene Ausbildung des Tubus 1 und/oder der Verschlusseinrichtung 16 ist denkbar und möglich, aber weniger bevorzugt.

Die Verschlussmantelöffnung 22 ist so groß ausgebildet, dass sie in einer entsprechenden Winkelstellung zwischen der Verschlusseinrichtung 16 und dem in den Hohlraum 17 der Verschlusseinrichtung 16 eingeschobenen Tubus 1 sowohl die Tubusmantelöffnung 9 als auch die Ligaturöffnung 5 frei gibt, also mit diesen in Deckung liegt. Die Verschlussmantelöffnung 22 erstreckt sich in Längsrichtung zwischen dem distalen Ende 31' und dem proximalen Ende 32'. Erfindungsgemäß sind zum Stützen der Mukosa während der Operation die Stege 23 vorgesehen. Diese sind in diesem Ausführungsbeispiel einstückig an der Verschlussmantelwand 21 angeformt. Darüber hinaus sind die Stege 23 entsprechend der Verschlussmantelwand 21 in Umfangsrichtung 33 gekrümmt. Die hier in diesem Ausführungsbeispiel beabstandet voneinander angeordneten und parallel verlaufenden Stege 23 erstrecken sich nur über einen ersten Abschnitt 22a der Verschlussmantelöffnung 22. Es bleibt ein zweiter Abschnitt 22b der Verschlussmantelöffnung 22 über die gesamte Längserstreckung der Verschlussmantelöffnung 22 zwischen distalem Ende-31' und proximalem Ende 32' vollkommen frei von den Stegen 23. Diese weisen somit jeweils ein freies Ende und ein an der Verschlussmantelwand 21 angeordnetes bzw. einstückig angeformtes Ende auf. Hierdurch ist eine insgesamt kamm- bzw. rechenartige Struktur mittels der Stege 23 geschaffen. Die Anzahl der Stege 23 kann je nach gewünschtem Abstand und Dichte der in der Mukosa anzubringenden Nähte variieren. Günstigerweise sind zwischen zwei und sechs, vorzugsweise zwischen drei und fünf Stege 23 vorgesehen. Im gezeigten Ausführungsbeispiel sind die Stege 23 ausschließlich an der Verschlussmantelwand 21 angeordnet. Dies muss aber nicht so sein. Es ist durchaus auch möglich, entsprechende Stege 23 an der Tubusmantelwand 2 bzw. in der Tubusmantelöffnung 9 anzuordnen. Sogar die Anordnung von Stegen 23 an Tubusmantelwand 2 und Verschlussmantelwand 21 ist denkbar, woraus sich eine Struktur mit zwei überlappenden Kämmen ergeben kann. Darüber hinaus kann bei entsprechender Operationstechnik gegebenenfalls auch auf die freien Enden der Stege 23 bzw. den Abschnitt 22b verzichtet werden. In diesem Fall queren die Stege 23 dann die gesamte Verschlussmantelöffnung 22 und/oder Tubusmantelöffnung 9.

Fig. 2 zeigt das proximale Ende der Einrichtung. Zu sehen ist der zum proximalen Ende 4 hin offene Hohlraum 5 des Tubus 1. In diesem können - wie an sich bekannt - Operationsinstrumente, Beleuchtungseinrichtungen, Absaugeinrichtungen u. dgl. in den inneren Hohlraum 5 des Tubus eingeführt werden. Einige Operationsinstrumente sowie günstige Formen der Beleuchtung sind beispielhaft in der EP 1 683 473 sowie in der EP 1 234 539 beschrieben und dargestellt.

Fig. 3 zeigt die Schließstellung der Einrichtung, in der sie zu Beginn der Operation in das Rektum des Patienten eingeführt wird. In dieser Stellung ist die Verschlusseinrichtung 16 so gegen den Tubus 1 verdreht, dass die geschlossenen Bereiche der Verschlussmantelwand 21 die Ligaturöffnung 10, wie auch die Tubusmantelöffnung 9 vollständig abdecken. Entsprechend ist die Verschlussmantelöffnung 22 durch die innenliegende Tubusmantelwand 2 verschlossen. Nach Einführen in das Rektum wird durch Verdrehen der Verschlusseinrichtung 16 gegen den Tubus 1 die in Fig. 4 dargestellte Öffnungsstellung eingestellt. Wann diese Stellung erreicht wird, ist mittels des Anschlages 24 der Verschlusseinrichtung 16 und entsprechenden Markierung an der Halterung 7 des Tubus 1 feststellbar. Mittels der Ultraschallsonde 12 kann nun die zu operierende Arterie gesucht werden. Ist diese gefunden und über die Ligaturöffnung 10 und die Tubusmantelöffnung 9 für das im inneren Hohlraum 5 des Tubus 1 geführte Operationsinstrument zugänglich, so können die in Fig. 4a schematisch gezeigten Nähte angebracht werden. 25 symbolisiert dabei die an sich bekannte HAL-Operation in der Ligaturöffnung 10. Die mittels der Nähte 26a bis 26e veranschaulichte Raffnaht wird hingegen durch die Tubusmantelöffnung 9 hindurch in der Mukosa angebracht. Hierzu wird zunächst mit der distal gelegenen Naht 26a begonnen. Anschließend werden mit dem selben durchgehenden Faden 27 die Nähte 26b, c, d und e nacheinander angebracht. Am proximalen Ende des Fadens 27 verbleibt anschließend der Überstand 28. Während dieses Teils der Operation stützen die in dieser Stellung über der Tubusmantelöffnung 9 liegenden Stege 23 die Mukosa ab, sodass diese nicht übermäßig weit durch die Tubusmantelöffnung 9 hindurch in den inneren Hohlraum 5 des Tubus 1 gedrückt werden kann. Ist ein in Fig. 4a gezeigter Operationsabschnitt fertig gestellt, so kann die Verschlusseinrichtung 16 weiter in Umfangsrichtung 33 gedreht werden, bis der Anschlag 24 der Verschlusseinrichtung 16 an der Basis des Handgriffs 8 anschlägt. Ist diese in Fig. 5 dargestellte Stellung erreicht, so sind die Stege 23 nicht mehr vor der Tubusmantelöffnung 19 angeordnet. Der zweite Abschnitt 22b der Verschlussmantelöffnung 22 ist in Deckung mit der Tubusmantelöffnung 9 gebracht. Fig. 5a zeigt die zu Fig. 4a entsprechende Ansicht aus dem Inneren des Hohlraums 5 des Tubus 1 auf die Mukosa bzw. die HAL-Operation 25 und die mittels des Fadens 27 gebildete Raffnaht. Nachdem die Stege 23 zurückgezogen sind, kann das proximale Ende 28 des Fadens 27 mit der distalen Naht 26a verknotet werden, wobei die proximale Naht 26e zur distalen Naht 26a geführt wird. Weitere HAL-Operationen bzw. Raffnähte können an anderen Arterien nach Drehen der gesamten Einrichtung im Rektum durchgeführt werden. Häufig sind drei oder mehrere Schleimhautvorfälle zu operieren.

Werden entgegen des gezeigten Beispiels die Nähte 26a bis 26e nicht mit einem durchgehenden Faden 27 sondern mit Einzelfäden angefertigt, so ist es - wie oben bereits angedeutet - auch denkbar, die Stege 23 so lang auszubilden, dass sie die gesamte Verschlussmantelöffnung 22 queren. Es entfällt dann auch der Schritt des Verdrehens aus der in Fig. 4 gezeigten Stellung in die in Fig. 5 gezeigte Stellung.

Der Tubus 1 der im Ausführungsbeispiel gezeigten Einrichtung kann aber nicht nur, wie anhand der Fig. 4a und 5a erläutert, für eine HAL-Operation oder das Anbringen einer Raffnaht benutzt werden. Es ist darüber hinaus auch möglich, einen Hämmorrhoidenprolaps durch Gummibandligatur zu behandeln. Diese Art der Operation ist an sich bekannt und in der EP 1 234 539 anhand der Fig. 12 erläutert, sodass auf die Operationstechnik an sich hier nicht weiter eingegangen werden muss. Allerdings wird darauf hingewiesen, dass bei dieser Operation auf die Verwendung der Verschlusseinrichtung 16 verzichtet werden kann. Der Tubus 1 wird direkt, also ohne Verschlusseinrichtung 16 in das Rektum des Patienten eingeführt. Die Operation kann dann durch die distale Öffnung 15 der Tubusmantelwand 2 hindurch erfolgen. Um das Einführen in das Rektum möglichst schmerzfrei und einfach zu gestalten, ist gemäß einem besonderen Aspekt der Erfindung jedoch vorgesehen, dass vor dem Einführen der Einrichtung in das Rektum ein Einschubkörper 29 in den inneren Hohlraum 5 des Tubus 1 eingeschoben wird. Das distale Ende des Einschubkörpers 29 ist dabei günstigerweise im Wesentlichen konisch geformt und steht im eingeschobenen Zustand distal über die Tubusmantelwand 2 vor.

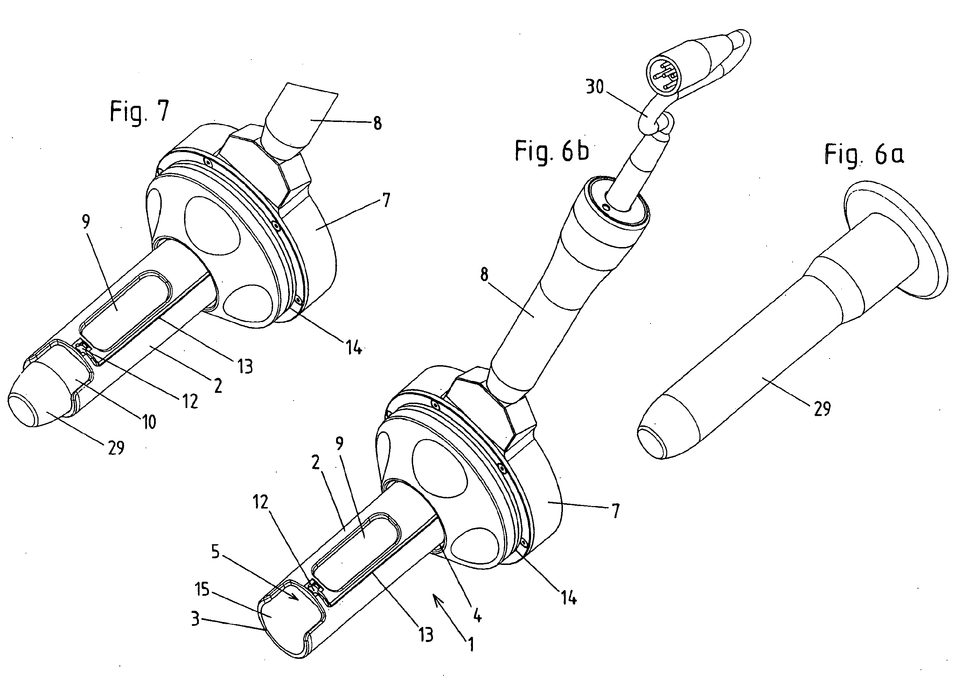

Zur Durchführung der Gummibandligatur wird der Einschubkörper 29 dann entfernt, bevor die zur Operation benötigten Instrumente in den Hohlraum 5 eingeführt werden. Die Fig. 6a bis 7 zeigen den Tubus 1 mit Einschubkörper 29. Zu sehen sind auch die Anschlusskabel 30 für den Ultraschallsensor 12 bzw. gegebenenfalls vorgesehene weitere Operationshilfsmittel der Einrichtung.

Die Dimensionierung der Einrichtung und der Öffnungen in Tubusmantelwand 2 und Verschlussmantelwand 21 liegen günstigerweise im Bereich des beim Stand der Technik bekannten. So beträgt die über die Halterung 7 vorstehende achsiale Länge des Tubus 1 vorzugsweise 60 bis 100mm. Die Längserstreckung der Tubusmantelöffnung 9 liegt günstigerweise zwischen 40 und 80mm, ihre Breite liegt günstigerweise zwischen 8 und 20mm.

Unterschiedliche Modifikationen der verschriebenen Ausführungsbeispiele sind denkbar und möglich, ohne den Bereich der Erfindung, wie er in den Ansprüchen definiert ist, zu verlassen.

### Legende zu den Hinweisziffern

- 1: Tubus
- 2: Tubusmantelwand
- 3: distales Ende der Tubusmantelwand
- 3': distales Ende der Tubusmantelöff- nung
- 4: proximales Ende der Tubusmantel- wand
- 4': proximales Ende der Tubus- mantelöffnung
- 5: innerer Hohlraum des Tubus
- 6: Längsachse
- 7: Halterung
- 8: Handgriff
- 9: Tubusmantelöffnung
- 10: Ligaturöffnung
- 11: Zwischensteg
- 12: Ultraschallsensor
- 13: Kanal
- 14: Führung
- 15: distale Öffnung der Tubusmantelwand
- 16: Verschlusseinrichtung
- 17: Hohlraum in Verschlussmantelwand
- 18: distaler Abschnitt der Verschlusseinrichtung
- 19: distale Öffnung der Verschlussmantelwand
- 20: Führungsabschnitt der Verschlusseinrichtung
- 21: Verschlussmantelwand
- 22: Verschlussmantelöffnung
- 22a: erster Abschnitt
- 22b: zweiter Abschnitt
- 23: Steg
- 24: Anschlag
- 25: HAL-Operation
- 26a: Naht der Raffnaht
- 26b: Naht der Raffnaht
- 26c: Naht der Raffnaht
- 26d: Naht der Raffnaht
- 26e: Naht der Raffnaht
- 27: Faden
- 28: proximales Ende des Fadens
- 29: Einschubkörper
- 30: Anschlusskabel
- 31: distales Ende der Verschlussmantelwand
- 31': distales Ende der Verschlussmantelöffnung
- 32: proximales Ende der Verschlussmantelwand
- 32': proximales Ende der Verschlussmantelöffnung
- 33: Umfangsrichtung

## Patentansprüche

1. Einrichtung zur Verwendung bei der Behandlung eines Hämorrhoidenprolaps, insbesondere durch HAL-Operation und/oder Anbringen einer Raffungsnaht und/oder einer Gummibandligatur, mit einem Tubus mit einer Tubusmantelwand und einer Verschlusseinrichtung mit einer einen Hohlraum umschließenden Verschlussmantelwand, wobei die Tubusmantelwand im Hohlraum der Verschlussmantelwand verschiebbar und/oder drehbar lagerbar ist und die Tubusmantelwand und die Verschlussmantelwand einzeln oder gemeinsam in ein Rektum eines Patienten einführbar sind, wobei die Tubusmantelwand zumindest eine Tubusmantelöffnung und die Verschlussmantelwand zumindest eine Verschlussmantelöffnung aufweisen und die Tubusmantelöffnung und die Verschlussmantelöffnung in zumindest einer Öffnungsstellung zumindest teilweise miteinander in Deckung bringbar sind, **dadurch gekennzeichnet, dass** zumindest ein an der Tubusmantelwand (2) und/oder an der Verschlussmantelwand (21) angeordneter Steg (23) vorgesehen ist, welcher in die Tubusmantelöffnung (9) und/oder in die Verschlussmantelöffnung (22) zumindest teilweise hineinragt.

2. Einrichtung nach Anspruch 1, **dadurch gekennzeichnet, dass** mehrere an der Tubusmantelwand (2) und/oder an der Verschlussmantelwand (21) angeordnete Stege (23) vorgesehen sind, welche in die Tubusmantelöffnung (9) und/oder in die Verschlussmantelöffnung (22) zumindest teilweise hineinragen.

3. Einrichtung nach Anspruch 1 oder 2, **dadurch gekennzeichnet, dass** die Tubusmantelwand (2) und/oder die Verschlussmantehnrand (21) zwischen einem distalen Ende (3,31) und einem proximalen Ende (4,32) längserstreckt ausgebildet ist (sind).

4. Einrichtung nach einem der Ansprüche 1 bis 3, **dadurch gekennzeichnet, dass** die Tubusmantelwand (2) und/oder die Verschlussmantelwand (21) abgesehen von darin vorgesehenen Öffnungen zumindest bereichsweise rotationssymmetrisch bezüglich einer Längsachse (6), vorzugsweise zylinder- oder kegel- oder kegelstumpfförmig, ausgebildet ist (sind).

5. Einrichtung nach einem der Ansprüche 1 bis 4, **dadurch gekennzeichnet, dass** die Tubusmantelöffnung (9) und/oder die Verschlussmantelöffnung (22) zwischen ihrem distalen Ende (3',31') und ihrem proximalen Ende (4',32') längserstreckt ist (sind) und/oder dass die Tubusmantelwand (2) einen zum proximalen Ende (4) hin offenen Hohlraum (5) zum Einführen von Operationsinstrumenten umschließt.

6. Einrichtung nach einem der Ansprüche 1 bis 5, **dadurch gekennzeichnet, dass** ein jeweiliger Steg (23) beabstandet vom distalen Ende (3',31') und vom proximalen Ende (4',32') der Tubusmantelöffnung (9) und/oder der Verschlussmantelöffnung (22) angeordnet ist.

7. Einrichtung nach einem der Ansprüche 1 bis 6, **dadurch gekennzeichnet, dass** im Falle mehrerer Stege (23) jeweils zwei benachbarte Stege (23) voneinander beabstandet, vorzugsweise parallel zueinander verlaufend, angeordnet sind.

8. Einrichtung nach einem der Ansprüche 1 bis 7, **dadurch gekennzeichnet, dass** ein jeweiliger Steg (23) längserstreckt ist und seine Längserstreckung im Wesentlichen senk recht zu einer Längsachse (6) der Tubusmantelwand (2) und/oder der Verschlussmantelwand (21) verlaufen.

9. Einrichtung nach einem der Ansprüche 1 bis 8, **dadurch gekennzeichnet, dass** ein jeweiliger Steg (23) in einer Umfangsrichtung (33) der Tubusmantelwand (2) oder der Verschlussmantelwand (21) wie diese gekrümmt ist und/oder dass ein jeweiliger Steg (23) einstückig an der Tubusmantelwand (2) und/oder der Verschlussmantelwand (21) angeformt ist.

10. Einrichtung nach einem der Ansprüche 1 bis 9, **dadurch gekennzeichnet, dass** der Steg (23) oder die Stege (23) sich nur über einen ersten Abschnitt (22a) der Tubusmantelöffnung (9) und/oder der Verschlussmantelöffnung (22) erstrecken und ein zweiter, vorzugsweise sich vom distalen Ende (3',31') zum proximalen Ende (4',32') der Tubusmantelöffnung (9) und/oder der Verschlussmantelöffnung (22) erstreckender, Abschnitt (22b) der Tubusmantelöffnung (9) und/oder der Verschlussmantelöffnung (22) frei von Stegen (23) ist.

11. Einrichtung nach einem der Ansprüche 1 bis 10, **dadurch gekennzeichnet, dass** ein jeweiliger Steg (23) jeweils ein freies Ende und ein an der Tubusmantelwand (2) und/oder der Verschlussmantelwand (21) angeordnetes Ende aufweist (aufweisen) und/oder dass die Tubusmantelwand (2) und/oder die Verschlussmantelwand (21) ein distal offenes Ende (15, 19) aufweist (aufweisen).

12. Einrichtung nach einem der Ansprüche 1 bis 11, **dadurch gekennzeichnet, dass** die Gestalt der Außenfläche der Tubusmantelwand (2) so exakt mit der Gestalt der Innenfläche der Verschlussmantelwand (21) übereinstimmt, dass bei Einführen der Tubusmantelwand (2) in den Hohlraum (17) der Verschlussmantelwand (21), bis auf das zum Einführen bzw. Drehen benötigte Spiel, ein Passsitz erzielbar ist.

13. Einrichtung nach einem der Ansprüche 1 bis 12, **dadurch gekennzeichnet, dass**, vorzugsweise an oder in der Tubusmantelwand (2), ein Ultraschallsensor (12) zur Lokalisierung einer Arterie vorgesehen ist und/oder dass die Tubusmantelwand (2) zusätzlich zurTubusmantelöffnung (9) eine davon durch einen Zwischensteg (11) getrennte Ligaturöffnung (10) aufweist.

14. Einrichtung nach Anspruch 13, **dadurch gekennzeichnet, dass** der Ultraschallsensor (12) auf dem Zwischensteg (11) angeordnet ist.

15. Einrichtung nach einem der Ansprüche 13 oder 14, **dadurch gekennzeichnet, dass** der Ultraschallsensor (12) dazu vorgesehen ist, eine Arterie im Bereich der Ligaturöffnung (10) oder der Tubusmantelöffnung (9) zu Lokalisieren.

## Claims

1. A device for use in the treatment of a haemorrhoid prolapse, in particular by HAL surgery and/or the attachment of a gathering suture and/or an elastic-band ligature, having a tube with a tube casing wall and a closure device with a closure casing wall which encloses a cavity, wherein the tube casing wall can be displaecably and/or rotatably mounted in the cavity of the closure casing wall and the tube casing wall and the closure casing wall are insertable, individually or together, into a rectum of a patient, wherein the tube casing wall has at least one tube casing opening and the closure casing wall has at least one closure casing opening and the tube casing opening and the closure casing opening can, in at least one open position, be brought into at least partial congruence with one another, **characterised in that** at least one arm (23), arranged on the tube casing wall (2) and/or on the closure casing wall (21) is provided, which arm (23) at least partially projects into the tube casing opening (9) and/or into the closure casing opening (22).

2. A device according to claim 1, **characterised in that** a plurality of arms (23) arranged on the tube casing wall (2) and/or on the closure casing wall (21) are provided, which at least partially project into the tube casing opening (9) and/or into the closure casing opening (22).

3. A device according to claim 1 or 2, **characterised in that** the tube casing wall (2) and/or the closure casing wall (21) is (are) elongate between a distal end (3, 31) and a proximal end (4, 32).

4. A device according to any one of claims 1 to 3, **characterised in that** at least regions of the tube casing wall (2) and/or the closure casing wall (21) are, with the exception of openings provided therein, rotationally symmetrical with respect to a longitudinal axis (6), are preferably in the form of a cylinder or a cone or a truncated cone.

5. A device according to any one of claims 1 to 4, **characterised in that** the tube casing opening (9) and/or the closure casing opening (22) is (are) elongate between their distal end (3', 31') and their proximal end (4', 32'), and/or **in that** the tube casing wall (2) encloses a cavity (5), open towards the proximal end (4), for the insertion of surgical instruments.

6. A device according to any one of claims 1 to 5, **characterised in that** a respective arm (23) is arranged at a distance from the distal end (3', 31') and from the proximal end (4', 32') of the tube casing opening (9) and/or the cloture casing opening (22).

7. A device according to any one of claims 1 to 6, **characterised in that** in the event of a plurality of arms (23), in each case two adjacent arms (23) are arranged at a distance from one another, preferably running parallel to one another.

8. A device according to any one of claims 1 to 7, **characterised in that** a respective arm (23) is elongate and its longitudinal extension runs substantially perpendicular to a longitudinal axis (6) of the tube casing wall (2) and/or the closure casing wall (21).

9. A device according to any one of claims 1 to 8, **characterised in that** a respective arm (23) is, in a circumferential direction (33) of the tube casing wall (2) or the closure casing wall (21), curved in the manner thereof, and/or **in that** a respective arm (23) is integrally formed on the tube casing wall (2) and/or the closure casing wall (21).

10. A device according to any one of claims 1 to 9, **characterised in that** the arm (23) or the arms (23) extend only over a first portion (22a) of the tube casing opening (9) and/or the closure casing opening (22), and a second portion (22b), preferably extending from the distal end (3', 31') to the proximal end (4', 32') of the tube casing opening (9) and/or the closure casing opening (22), of the tube casing opening (9) and/or the closure casing opening (22) is devoid of arms (23).

11. A device according to any one of claims 1 to 10, **characterised in that** a respective arm (23) has in each case a free end and an end arranged on the tube casing wall (2) and/or the closure casing wall (21), and/or **in that** the tube casing wall (2) and/or the closure casing wall (21) has (have) a distally open end (15, 19).

12. A device according to any one of claims 1 to 11, **characterised in that** the form of the outer surface of the tube casing wall (2) corresponds to the form of the inner surface of the closure casing wall (21) so exactly that a snug fit can be achieved upon insertion of the tube casing wall (2) into the cavity (17) of the closure casing wall (21) except for the play required for insertion or rotation as the case may be

13. A device according to any one of claims 1 to 12, **characterised in that** an ultrasonic sensor (12) for localization of an artery is provided, preferably on or in the tube casing wall (2), and/or **in that** in addition to the tube casing opening (9), the tube casing wall (2) has a ligature opening (10) separated from the tube casing opening (9) by an intermediate arm (11).

14. A device according to claim 13, **characterised in that** the ultrasonic sensor (12) is arranged on the intermediate arm (11).

15. A device according to one of claims 13 and 14, **characterised in that** the ultrasonic sensor (12) is provided for artery localisation in the region of the ligature opening (10) or the tube casing opening (9).

## Revendications

1. Dispositif destiné au traitement d'un prolapsus hémorroïdaire, notamment par ligature d'artère hémorroïdaire et/ou exécution d'une suture en bourse et/ou d'une ligature élastique, comprenant un tube avec une paroi d'enveloppe de tube et un dispositif d'obturation avec une paroi d'enveloppe d'obturation entourant une cavité, la paroi d'enveloppe de tube étant logée de manière à pouvoir coulisser et/ou tourner dans la cavité de la paroi d'enveloppe d'obturation, la paroi d'enveloppe de tube ainsi que la paroi d'enveloppe d'obturation pouvant être introduites individuellement ou conjointement dans le rectum d'un patient, la paroi d'enveloppe de tube présentant au moins une ouverture d'enveloppe de tube et la paroi d'enveloppe d'obturation au moins une ouverture d'enveloppe d'obturation, et l'ouverture d'enveloppe de tube et l'ouverture d'enveloppe d'obturation pouvant être amenées à se chevaucher au moins partiellement dans au moins une position d'ouverture, **caractérisé en ce qu'**il est prévu au moins une nervure (23) disposée sur la paroi d'enveloppe de tube (2) et/ou sur la paroi d'enveloppe d'obturation (21), laquelle pénètre au moins partiellement dans l'ouverture d'enveloppe de tube (9) et/ou dans l'ouverture d'enveloppe d'obturation (22).

2. Dispositif selon la revendication 1, **caractérisé en ce que** sont prévues plusieurs nervures (23) disposées sur la paroi d'enveloppe de tube (2) et/ou sur la paroi d'enveloppe d'obturation (21), lesquelles pénètrent au moins partiellement dans l'ouverture d'enveloppe de tube (9) et/ou dans l'ouverture d'enveloppe d'obturation (22).

3. Dispositif selon la revendication 1 ou la revendication 2, **caractérisé en ce que** la paroi d'enveloppe de tube (2) et/ou la paroi d'enveloppe d'obturation (21) sont étirées en longueur entre une extrémité distale (3, 31) et une extrémité proximale (4, 32).

4. Dispositif selon l'une des revendications 1 à 3, **caractérisé en ce que** la paroi d'enveloppe de tube (2) et/ou la paroi d'enveloppe d'obturation (21), compte non tenu des ouvertures prévues en elles, sont au moins partiellement à symétrie de rotation par rapport à un axe longitudinal (6), et de préférence de forme cylindrique, conique ou tronconique.

5. Dispositif selon l'une des revendications 1 à 4, **caractérisé en ce que** l'ouverture d'enveloppe de tube (9) et/ou l'ouverture d'enveloppe d'obturation (22) sont étirées en longueur entre leur extrémité distale (3', 31') et leur extrémité proximale (4', 32'), et/ou **en ce que** la paroi d'enveloppe de tube (2) entoure une cavité (5) ouverte vers l'extrémité proximale (4) pour l'insertion d'instruments opératoires.

6. Dispositif selon l'une des revendications 1 à 5, **caractérisé en ce qu'**une nervure (23) est disposée à intervalle de l'extrémité distale (3', 31') et de l'extrémité proximale (4', 32') de l'ouverture d'enveloppe de tube (9) et/ou de l'ouverture d'enveloppe d'obturation (22).

7. Dispositif selon l'une des revendications 1 à 6, **caractérisé en ce que**, dans le cas de plusieurs nervures (23), deux nervures (23) contiguës sont disposées à intervalle l'une de l'autre, de préférence parallèlement l'une à l'autre.

8. Dispositif selon l'une des revendications 1 à 7, **caractérisé en ce qu'**une nervure (23) est étirée en longueur et que son extension longitudinale est sensiblement perpendiculaire à un axe longitudinal (6) de la paroi d'enveloppe de tube (2) et/ou de la paroi d'enveloppe d'obturation (21).

9. Dispositif selon l'une des revendications 1 à 8, **caractérisé en ce que**, dans une direction périphérique (33) de la paroi d'enveloppe de tube (2) ou de la paroi d'enveloppe d'obturation (21), une nervure (23) est cintrée identiquement à celles-ci, et/ou **en ce qu'**une nervure (23) est formée d'un seul tenant sur la paroi d'enveloppe de tube (2) et/ou la paroi d'enveloppe d'obturation (21).

10. Dispositif selon l'une des revendications 1 à 9, **caractérisé en ce que** la nervure (23) ou les nervures (23) ne s'étendent que sur une première partie (22a) de l'ouverture d'enveloppe de tube (9) et/ou de l'ouverture d'enveloppe d'obturation (22), et **en ce qu'**une deuxième partie (22b) de l'ouverture d'enveloppe de tube (9) et/ou de l'ouverture d'enveloppe d'obturation (22), laquelle s'étend préférentiellement de l'extrémité distale (3', 31') à l'extrémité proximale (4', 32') de l'ouverture d'enveloppe de tube (9) et/ou de l'ouverture d'enveloppe d'obturation (22), est libre de nervures (23).

11. Dispositif selon l'une des revendications 1 à 10, **caractérisé en ce qu'**une nervure (23) présente une extrémité libre et une extrémité disposée contre la paroi d'enveloppe de tube (2) et/ou la paroi d'enveloppe d'obturation (21), et/ou en ce que la paroi d'enveloppe de tube (2) et/ou la paroi d'enveloppe d'obturation (21) présentent une extrémité distale ouverte (15, 19).

12. Dispositif selon l'une des revendications 1 à 11, **caractérisé en ce que** la forme de la surface extérieure de la paroi d'enveloppe de tube (2) correspond si précisément à la forme de la surface intérieure de la paroi d'enveloppe d'obturation (21) qu'il est possible d'obtenir un ajustement fin lors de l'introduction de la paroi d'enveloppe de tube (2) dans la cavité (17) de la paroi d'enveloppe d'obturation (21), compte non tenu du jeu exigé pour l'introduction ou la rotation.

13. Dispositif selon l'une des revendications 1 à 12, **caractérisé en ce que**, de préférence sur ou dans la paroi d'enveloppe de tube (2), il est prévu un capteur à ultrasons (12) pour la localisation d'une artère, et/ou **en ce que** la paroi d'enveloppe de tube (2) présente en plus de l'ouverture d'enveloppe de tube (9) une ouverture pour ligature (10) séparée de la précédente par une nervure intercalaire (11).

14. Dispositif selon la revendication 13, **caractérisé en ce que** le capteur à ultrasons (12) est disposé sur la nervure intercalaire (11).

15. Dispositif selon l'une des revendications 13 ou 14, **caractérisé en ce que** le capteur à ultrasons (12) est prévu pour localiser une artère dans la zone de l'ouverture pour ligature (10) ou de l'ouverture d'enveloppe de tube (9).
